# EUROPÄISCHE PATENTSCHRIFT

(11) **EP 1 524 982 B1**
(45) Veröffentlichungstag und Bekanntmachung des Hinweises auf die Patenterteilung: **24.02.2010**
(21) Anmeldenummer: 03784081.6
(22) Anmeldetag: 25.07.2003
(51) Int. Cl.: A61K 31/5513, A61K 31/465

(54) **MEDIKAMENT UND VERFAHREN ZUR VERRINGERUNG DES ALKOHOL-UND/ODER TABAKKONSUMS**
MEDICAMENT AND METHOD FOR REDUCING ALCOHOL AND/OR TOBACCO CONSUMPTION
MEDICAMENT ET PROCEDE POUR LIMITER LA CONSOMMATION D'ALCOOL ET/OU DE TABAC

(30) Priorität: 03.08.2002 DE 10235556
(43) Veröffentlichungstag der Anmeldung: 27.04.2005
(73) Patentinhaber: HF ARZNEIMITTELFORSCHUNG GmbH, 59368 Werne (DE)
(72) Erfinder: MOORMANN, Joachim, 59368 Werne (DE); OPITZ, Klaus, 48157 Münster (DE); MUCKE, Hermann, A-1160 Wien (AT)
(74) Vertreter: Flaccus, Rolf-Dieter
(86) Internationale Anmeldenummer: PCT/EP2003/008236
(87) Internationale Veröffentlichungsnummer: WO 2004/014393

(56) Entgegenhaltungen:
- WO-A-00/38686
- WO-A-97/47304
- DE-C- 4 301 782
- US-A- 5 589 475
- OPITZ K: "Tobacco dependence and remedies for withdrawal" PHARMAZEUTISCHE ZEITUNG 1996 GERMANY, Bd. 141, Nr. 26, 1996, Seiten 46-48, XP001155945 ISSN: 0031-7136

## Beschreibung

Gegenstand der vorliegenden Erfindung ist es, ein Medikament zur Therapie des Suchtverlangens bereit zu stellen, das eine getrennte oder gleichzeitige Reduktion des gemeinsam auftretenden Alkohol- und Nikotinabusus ermöglicht, unter besonderer Berücksichtigung des den Rückfall begünstigenden Durchbruchsverlangens.

Die vorliegende Erfindung betrifft insbesondere ein Medikament zur Therapie des Suchtverlangens, das aus zwei unterschiedlichen Darreichungsformen besteht, um eine verbesserte Therapie des Suchtverlangens bei Alkohol- und/oder Nikotinabusus, unter besonderer Berücksichtigung des den Rückfall begünstigenden Durchbruchsverlangens, zu ermöglichen.

Aufgabe der vorliegenden Erfindung ist es, ein Medikament zur Therapie des Suchtverlangens bereit zu stellen, das eine getrennte oder gleichzeitige Reduktion des gemeinsam auftretenden Alkohol- und Nikotinabusus ermöglicht, unter besonderer Berücksichtigung des den Rückfall begünstigenden Durchbruchsverlangens, dessen Anwendung die beruflichen oder sozialen Aktivitäten des Patienten nicht gravierend beeinträchtigt.

In neuerer Zeit hat sich die Pharmakologie verstärkt mit dem Symptomkomplex des Substanzverlangens befasst und eine Erklärung für zahlreiche damit in Zusammenhang stehende und bisher nicht befriedigend zu erklärende Verhaltensphänomene im molekularen Bereich geboten. Neurotransmitter, deren Bindung an neuronale Rezeptoren und die als Folge dieser Wechselwirkung bewirkte Modulation der Reizleitung gelten inzwischen als Basis für eine Erklärung des Suchtverhaltens.

Längere Zeit hindurch wurde dem dopaminergen System bei den pharmakologischen Modellvorstellungen des Suchtverhaltens fast ausschließliche Bedeutung beigemessen. Es wurde sogar - in Anlehnung an das "Serotonin-Mangelsyndrom", mit dem Depression und Zwangshandlungen erklärt werden - das Modell des "Dopamin-Mangelsyndroms" als hypothetische generalisierte Grundlage suchtbetonten Verhaltens im weiteren Sinne, das heißt auch von Formen des Verhaltens, die nicht mit Substanzkonsum verbunden sind, postuliert. Dem adrenergen und dem serotonergen Reizleitungssystem wurde nur sekundäre Bedeutung beigemessen, vor allem im Zusammenhang mit Depression im Suchtverhalten.

Hinsichtlich des Verlangens nach Alkohol- und Tabakprodukten wurde die die Bedeutung der adrenergen und serotonergen Reizleitungssysteme unterordnende Sicht in den letzten Jahren jedoch erheblich erweitert. Einen wesentlichen Teil des gründlicheren Verständnisses verdanken wir der Würdigung der Rolle der präsynaptisch lokalisierten nikotinischen Rezeptoren, deren natürlicher Ligand der Neurotransmitter Acetylcholin ist. Im cholinergen System dienen die präsynaptisch lokalisierten Acetylcholinrezeptoren als Autorezeptoren, d.h. sie blockieren die weitere Freisetzung von Acetylcholin, sobald dessen Konzentration in der Synapse einen Grenzwert erreicht. Nikotinische Autorezeptoren finden sich jedoch auch in den dopaminergen, adrenergen und serotonergen Reizleitungssystemen und modulieren dort in vergleichbarer Weise die Freisetzung der jeweiligen Neurotransmitter, sobald Acetylcholin, Nikotin oder ein anderer Ligand an sie bindet.

Neuronale nikotinische Rezeptoren koppeln also das cholinerge Reizleitungssystem mit den Reizleitungssystemen, die Dopamin, Norepinephrin oder Serotonin als Botenstoffe verwenden. Daher erscheint es plausibel anzunehmen, dass eine Erhöhung des cholinergen Tonus, wie sie durch Verabreichung von Medikamenten, welche die Konzentration von Acetylcholin entweder durch Steigerung der Ausschüttung oder durch Hemmung des abbauenden Enzyms Acetylcholinesterase erhöhen, bewirkt werden kann, auf dem Umweg über nikotinische Rezeptoren auch Auswirkungen auf die Stimmungslage und das Verlangen nach Alkohol und/oder Nikotin haben sollte.

Die Rolle neuronaler nikotinischer Rezeptoren als Schaltstelle zwischen Reizleitungssystemen, die mit unterschiedlichen Neurotransmittern arbeiten, ermöglicht auch eine Neubewertung der bekannten Tatsache, dass Alkoholiker sowie so genannten "Risikotrinker", die zwar nicht alle Kriterien der Alkoholabhäagigkeit nach den derzeit gültigen Schemata der "International Classification of Diseases" (ICD-10) erfüllen, aber die von der World Health Organization festgesetzten Grenzwerte für Alkoholkonsum dauerhaft überschreiten, fast immer gleichzeitig starke Raucher sind. Nach dem derzeitigen Stand der Wissenschaft stellen Alkohol- und/oder Nikotinabusus zu einem erheblichen Teil Auswirkungen einer gemeinsamen, fundamentalen Fehlsteuerung neuronaler nikotinischer Rezeptoren dar.

Trotz der gemeinsamen Auswirkungen auf die neuronalen nikotinischen Rezeptoren infolge von Alkohol- und/oder Nikotinabusus ist eine direkte und nachhaltige gegenseitige Substitution von Alkohol und Nikotin weder im Tiermodell noch im menschlichen Suchtverhalten nachzuweisen. Die pharmakologische Gabe von Nikotin, d. h. beispielsweise mittels transdermaler Gabe, als Kaugummi oder mittels eines Inhalators, beeinflusst gleichzeitig bestehenden Alkoholabusus im Allgemeinen nicht nennenswert. Andererseits ist allgemein bekannt, dass Alkoholentzug ein gleichzeitig bestehendes Rauchverhalten sowohl positiv als auch negativ beeinflussen kann.

Eine Erklärung für dieses Phänomen ist, dass nikotinische Rezeptoren nicht als einfache "Ein-Aus-Schalter" funktionieren, sondern aufgrund ihrer molekularen Struktur vielmehr der so genannten allosterischen Modulation unterliegen. Unter allosterischer Modulation ist zu verstehen, dass das Ansprechen auf die Bindung eines Liganden von der momentan gegebenen Konfiguration des Rezeptors abhängt, die wiederum durch an andere Stellen bindende Liganden beeinflusst wird.

Zudem ist bei beiden Formen der Sucht die derzeit übliche pharmakologische Rückfallprävention, wie sie im Rauchentzug durch Nikotinpflaster oder Nikotin-Kaugummi bzw. durch Acamprosat oder Naltrexon im Alkoholentzug geübt wird, äußerst mangelhaft. Rückfälle in den Alkohol- oder Nikotinkonsum bzw. in den gemeinsamen Abusus sind in den ersten Wochen und Monaten nach dem Entzug die Regel. Typischerweise erfolgen die Rückfälle im Rahmen eines so genannten "Durchbruchsverhaltens", worunter das Nachgeben gegenüber einem plötzlich auftretenden massiven Substanzverlangen vor dem Hintergrund eines sonst beherrschbaren Verlangens zu verstehen ist. Dieses Durchbruchsverlangen wird durch Stress, soziale Reize, Anblick oder Geruch alkoholischer Getränke bzw. Zigarettenrauch oder dergleichen ausgelöst.

Es erscheint somit auf den ersten Blick nahe liegend, dieses im Rahmen einer pharmakologisch unterstützten Abstinenz auftretende akute Verlangen durch eine schnelle Zufuhr einer zusätzlichen Dosis des betreffenden rückfallverzögernden Mittels zu bekämpfen, beispielsweise durch die bedarfsweise Verwendung eines nikotinhaltigen Kaugummis zusätzlich zu der transdermal verabreichten Basisdosis an Nikotin. Dem Fachmann ist jedoch bekannt, dass dieser Weg keineswegs erfolgversprechend, sondern vielmehr potentiell abhängigkeitsverstärkend und unter Umständen sogar gefährlich ist. So desensibilisiert schnell in das Zentralnervensystem eingebrachtes Nikotin die dortigen nikotinischen Rezeptoren innerhalb von Sekunden bis Minuten weitgehend. Daher ist Rauchen, wodurch Nikotin schneller in das Gehirn gelangt als es auf intravenösem Weg möglich wäre, suchterzeugend, wohingegen transdermal verabreichtes Nikotin zu einem weit langsameren Anstieg der Nikotinkonzentration im Gehirn führt und kein vergleichbar hohes Abhängigkeitspotential hat.

Die auf den ersten Blick nahe liegende therapeutische Vorgehensweise würde daher die Abhängigkeit von Tabakprodukten nur durch eine Abhängigkeit von einem nikotinhaltigen Entzugsmittel ersetzen. Sinngemäß ähnliche überlegungen lassen sich auch für die Therapie des Alkoholverlangens anstellen.

In WO 00 38686 A1 wird eine Zusammensetzung für die kontrollierte Abgabe von Galanthamin beschrieben. Als bevorzugte Darreichungsformen werden solche genannt, bei denen ein Teil des Galanthamins in einer schnell freisetzenden Form enthalten ist, z. B. in Form von Minitabletten mit schneller Freisetzung. Beispielsweise kann diese Darreichungsform in Form einer oral zu verabreichenden Kapsel vorliegen, in der ein Teil der Galanthamindosis in Form von Pellets mit kontrollierter Freisetzung und der andere Teil der Galanthamindosis in Form von schnell freisetzenden Tabletten enthalten ist. Durch die kombinierte Anwesenheit unterschiedlich schnell freisetzender Wirkstoffzubereitungen innerhalb derselben Darreichungsform soll die Einstellung einer gewünschten Wirkstoffkinetik ermöglicht werden.

K. OPITZ ("Tabakabhängigkeit und Hilfsmittel zur Entwöhnung"; Pharmazeutische Zeitung 1996, Bd. 141, Nr. 26, S. 46-48; XP001155945) diskutiert verschiedene Möglichkeiten der Nikotinsubstitution zur Tabakentwöhnung. Die transdermale Verabreichung mittels Nikotinpflaster wird aufgrund der relativ langsamen Anflutung als optimal angesehen. Da schnell freisetzende, bukkale Nikotin-Darreichungsformen wie z. B. Nikotinkaugummi zur Aufrechterhaltung der Konditionierung der Raucher beitragen, wird deren Verwendung als nachteilig angesehen. Stattdessen wird es als wünschenswert angesehen, die Nikotinsubstitution ausschließlich durch transdermale Verabreichung mit ausreichend hoher Dosierung durchzuführen.

DE 43 01 783 C1 betrifft die Verwendung von Galanthamin zur Behandlung der Nikotinabhängigkeit, wobei der Wirkstoff auf kontinuierliche und kontrollierte Weise freigesetzt wird, z. B. oral, transdermal oder anderweitig parenteral. Nach transdermaler Verabreichung von Galanthamin wurde bei Rauchern eine Verminderung des Verlangens nach Zigaretten beobachtet.

WO 97 47304 A1 betrifft eine schnell lösliche Galanthaminhydrobromid-Tablette zur oralen Verabreichung. Durch Beimischung von Zerfallshilfsmitteln wie mikrokristalliner Zellulose soll im Unterschied zu bereits früher bekannten Galanthamintabletten eine schneller und reproduzierbare Auflösung erreicht werden.

US 5 589 475 A beschreibt die Verwendung von Cholinesterasehemmern, insbesondere Galanthamin, zur Verminderung von Nebenwirkungen, die durch Benzodiazepine verursacht werden. Galanthamin wird als Injektionslösung oder als orale, feste oder flüssige Darreichungsform verabreicht, beispielsweise als Tablette, Kapsel, Lösung oder Suspension.

Obwohl eine gemeinsame pharmakologische Therapie des Alkohol- und/oder Nikotinabusus auf dem Weg der Beeinflussung nikotinischer Rezeptoren von gesundheitlichen Standpunkt aus äußerst wünschenswert wäre, existiert eine solche Therapie bislang nicht. Insbesondere hat das "Durchbruchsverlangen" nach Alkohol und/oder Nikotin bisher keine auch nur annähernd adäquate pharmakologische Therapie erfahren.

Es wurde nunmehr überraschend und nach dem oben Gesagten für den Fachmann in keiner Weise vorhersehbar festgestellt, dass eine Zweikomponenten - Therapie des Alkohol- und/oder Nikotinverlangens dennoch möglich ist, und zwar unter der Voraussetzung, dass eine permanente Behandlung mittels einer pharmazeutischen Darreichungsform, welche Galanthamin oder eines seiner pharmakologisch akzeptablen Salze kontinuierlich abgibt, bei Auftreten starken Durchbruchaverlangens durch Verabreichung von Galanthamin oder einem seiner pharmakologisch akzeptablen Salze mittels einer Darreichungsform ergänzt wird, die ein schnelles Eintreten von Galanthamin oder einem seiner pharmakologisch akzeptablen Salze in das Zentralnervensystem ermöglicht.

Ein Ziel der vorliegenden Erfindung war daher die Bereitstellung eines Medikaments zur Therapie der Alkohol- und/oder Tabaksucht, insbesondere für die Durchführung der Zwei-Komponenten-Therapie zur Behandlung des Alkohol- und/oder Nikotinverlangens.

Das erfindungsgemäße Medikament zur Verwendung in einem Verfahren zur Behandlung des Suchtverlangens besteht aus einer Kombination von zwei Darreichungsformen, wobei die eine Darreichungsform ein transdermales therapeutisches System ist, das zumindest einen aus Galanthamin und pharmakologisch akzeptablen Salzen des Galanthamins bestehenden Gruppe ausgewählten Modulator nikotinischer Rezeptoren kontinuierlich abgibt, und die andere Darreichungsform, die unabhängig von der erstgenannten Darreichungsform verabreichbar ist, ein schnelles Eintreten von Galanthamin oder einem seiner pharmakologisch akzeptablen Salze in das Zentralnervensystem ermöglicht, wobei die einen schnellen Eintritt von Galanthamin oder einem pharmakologisch akzeptablen Salz des Galanthamins in das Zentralnervensystem ermöglichende Darreichungsform aus der Gruppe ausgewählt ist, die aus festen, im Speichel schnell löslichen, biokompatiblen Matrices, buccalen Lösungen sowie Sprüh- und Tropflösungen besteht.

Die den genannten Modulator nikotinischer Rezeptoren kontinuierlich abgebende Darreichungsform dient zur Therapie des Basisverlangens nach Alkohol und/oder Nikotin. Die ein schnelles Eintreten von Galanthamin oder seinen pharmakologisch akzeptablen Salzen ermöglichende Darreichungsform ermöglicht eine Therapie des trotz der Basistherapie plötzlich auftretenden Verlangens nach Alkohol und/oder Nikotin.

Der Modulator nikotinischer Rezeptoren in der diesen Modulator oder diese Modulatoren kontinuierlich abgebenden Darreichungsform wird vorzugsweise aus der Gruppe ausgewählt, die aus Galanthamin, den pharmakologisch akzeptablen Salzen des Galanthamins, Nikotin und den pharmakologisch akzeptablen Salzen des Nikotins besteht. Besonders bevorzugt wird Galanthamin für die den Modulator kontinuierlich abgebende Darreichungsform verwendet.

Die den Modulator oder die Modulatoren nikotinischer Rezeptoren kontinuierlich freisetzende Darreichungsform kann ein subkutanes Implantat oder ein intramuskulär injizierbare Präparat sein, die einen lang anhaltende Depoteffekt haben. Als intramuskulär injizierbare Präparate mit lang anhaltendem Depoteffekt kommen beispielsweise Suspensionen aus den Modulator oder die Modulatoren nikotinischer Rezeptoren enthaltenden Mikrokapseln in Betracht. Besonders bevorzugte Darreichungsformen für die kontinuierliche Abgabe sind transdermal therapeutische Systeme.

Die Abgaberate der den Modulator oder die Modulatoren nikotinischer Rezeptoren kontinuierlich freisetzende Darreichungsform liegt zwischen 10 mg und 25 mg Galanthamin oder einem der pharmakologisch akzeptablen Salze des Galanthamins bzw. zwischen 5 mg und 50 mg Nikotin oder einem der pharmakologisch akzeptablen Salze des Nikotins pro Tag.

Die Darreichungsformen, die ein schnelles Eintreten von Galanthamin oder seinen pharmakologisch akzeptablen Salzen in das Zentralnervensystem ermöglichen, enthalten zwischen 1 mg und 5 mg Galanthamin oder zumindest eines pharmakologisch unbedenklichen Salzes des Galanthamins.

Als Darreichungsformen, die ein schnelles Eintreten von Galanthamin oder seinen pharmakologisch akzeptablen Salzen in das Zentralnervensystem ermöglichen, kommen feste, im Speichel schnell lösliche, biokompatible Matrices etwa in Form einer Briefmarke in Betracht oder Lösungen, die in die Nasenhöhle eingetropft oder eingesprüht bzw. in der Mundhöhle behalten werden.

Die pernasal oder buccal zu verabreichenden Lösungen können in Form eines flexiblen, zwischen 1 und 5 ml fassenden Kunststoffbehältnisses dargereicht werden, wobei die Kunststoffbehhältnisse für die in die Nase einzusprühenden oder einzutropfenden Formulierungen mit entsprechend geformten Düsen versehen sind.

Erfingdungsgemäß wird die Kombinationstherapie zur Behandlung des Suchtverlangens bevorzugt derart gestaltet, dass die Therapie des Basisverlangens nach Alkohol und/oder Nikotin durch Erzeugung einer möglichst gleichmäßigen Galanthamin-Konzentration im Zeatralaerveasystem mittels einer Galanthamin langsam und kontinuierlich abgebenden Darreichungsform durchgeführt wird. Das bei einer derartigen Basistherapie dennoch plötzlich auftretende Verlangen nach Alkohol und/oder Nikotin wird erfindungsgemäß ausschließlich mit Galanthamin oder einem seiner pharmakologisch akzeptablen Salze therapiert, und zwar derart, dass eine schnelle Resorption des Galanthamins oder seines pharmakologisch akzeptablen Salzes durch die Schleimhaut der Mundhöhle oder Nase möglich ist.

Galanthamin (4a,5,9,11,12-Hexahydro-3-methoxy-11-methyl[6H]-benzofuro-[3a,3,2ef][2]beazazepin-6-ol) ist in Form von Tabletten und einer Trinklösung unter dem Handelsnamen Reminyl® zur Therapie der Alzheimer'schen Demenz zugelassen. Galanthamin moduliert neuronale nikotinische Rezeptoren durch zwei verschiedene Mechanismen: indirekt durch eine Erhöhung der synaptischen Konzentration von Acetylcholin mittels Hemmung der Acetylcholiaesterase, und direkt als so genannter allosterisch potenzierender Ligand (APL).

Die Verwendung von Galanthamin oder seiner pharmakologisch akzeptablen Säureadditionssalze als Mittel zur Bekämpfung eines Substanzverlangens ist dem Grunde nach bekannt. Die Verwendung zur Therapie des Alkoholabusus wird in der Patentschrift DE 4010079 offenbart. Galanthamin enthaltene transdermale therapeutische Systeme sind aus dem Patent DE 4301783 ebenfalls bekannt. Ein solches transdermales therapeutisches System, als einzige Therapie bei rückfälligen Alkoholikern eingesetzt, verringerte in einer klinischen Studie den Alkoholkonsum im Rückfall, beschleunigte jedoch das Eintreten des Rückfalls im Vergleich zum Placebo. Daraus geht hervor, dass die ausschließliche Verwendung von transdermal verabreichtem Galanthamin bei solchen Patienten keine optimale Therapielösung darstellt.

Die Verwendung von Galanthamin oder seiner pharmakologisch akzeptablen Säureadditionssalze zur Bekämpfung des Nikotinkonsums wird in dem Patent DE 4301782 beansprucht. Die zuvor angesprochene klinische Studie ergab bei den Probanden, die auch Raucher waren, Hinweise auf eine allerdings erst nach mehreren Wochen der ausschließlichen Therapie mit transdermal verabreichtem Galanthamin eintretende, relativ geringfügige Verringerung des Zigarettenkonsums. Wiederum scheint die alleinige Verwendung von transdermal verabreichtem Galanthamin nicht optimal zur Erreichung des Therapieziels.

In keinem der angesprochenen Dokumente ist die Verwendung von Galanthamin in schnell gehirngängiger Form zur Vermeidung oder Behandlung des Durchbruchsverlangens vor dem Hintergrund einer Dauertherapie mit Galanthamin oder einer anderen das Alkoholverlangen modifizierenden Substanz erwähnt. Im Gegenteil, Galanthamin wird in diesen Dokumenten ausschließlich unter Betonung auf seine Eignung für die Dauertherapie, insbesondere auch unter Verwendung von Darreichungsformen, die eine verzögerte Freisetzung bewirken, diskutiert und beansprucht.

In der therapeutischen Praxis wird das erfindungsgemäße Verfahren zur Therapie des substanzverlangens derart angewandt, dass die zu behandelnde Person, welche entweder klinisch alkoholabhängig mit bestehendem Alkoholkonsum, ein Alkoholiker unter Entzugstberapie oder nach deren Abschluss, oder aber Alkoholabusus im Sinne der WHO-Kriterien betreibend sein kann, wobei die Personen aus jeder dieser Gruppen auch Tabakprodukte konsumieren können, eine Basistherapie mit einem nikotinischen Agonisten erhält, unabhängig davon, ob die betreffende Person bei Therapiebeginn Tabakprodukte konsumiert oder nicht. Die Basistherapie erfolgt bevorzugt durch ein Galanthamin enthaltendes transdermales therapeutisches System, das pro Tag zwischen 10 mg und 25 mg Galanthamin abgibt, kann aber auch durch subkutane Implantate oder intramuskulär injizierbare Präparate mit lang anhaltendem Depoteffekt, die eine entsprechende Freisetzungsrate aufweisen, erfolgen. Injizierbare suspensionen von mikroverkapseltem Wirkstoff sind dem Fachmann bekannt, insbesondere in der Anwendung mit Psychopharmaka, beispielsweise durch die mikropartikulären Formulierungen gemäß US Patent 6 264 987 oder der WO 00/35423. Ebenso sind biokompatible, abbaubare subkutane Implantate bekannt, beispielsweise aus dem US Patent 6 312 708.

Alternativ dazu kann die Basistherapie auch mit handelsüblichen, Nikotin enthaltenden transdermalen therapeutischen Systemen durchgeführt werden. Transdermale therapeutische Systeme, die Nikotin als einzigen Wirkstoff zur Bekämpfung des Tabakkonsums enthalten, sind beispielsweise in dem deutschen Patent DE 3629304 oder im US Patent 4 597 961 beschrieben. Im erfindungsgemäßen Sinne verwendbar sind auch die in der WO 01/80837 beanspruchen transdermalen Systeme, die Kombinationen von Nikotin oder dessen Salzen, wahlweise unter zusätzlicher Verwendung nikotinisch wirkender Substanzen wie Lobelin oder Succinylcholin, mit antidepressiv wirkenden Verbindungen enthalten.

Falls die derart behandelte Person trotz dieser Basistherapie ein plötzlich einsetzendes Verlangen nach Alkohol und/oder Tabakprodukten verspürt, oder unmittelbar im Begriff ist, sich in eine Situation zu begeben, in der das Auftreten eines Verlangens nach Alkohol und/oder Tabakprodukten erfahrungsgemäß zu erwarten ist, beispielsweise bei der Teilnahme an Veranstaltungen, bei denen getrunken und geraucht wird, erfolgt eine Selbstverabreichung von 1 bis 5 mg Galanthamin in einer Form, die durch transmukosale Resorption eine innerhalb von Minuten einsetzende und 2 bis 3 Stunden wirksame Absicherung gegen das Durchbruchsverlangen bewerkstelligt. Wesentlich ist dabei, dass diese schnell wirksame Medikation einfach mitzuführen ist und ihre Verabreichung schnell, schmerzlos, sozial unauffällig und ohne Zuhilfenahme von Hilfsmitteln erfolgen kann.

Erfingdungsgemäß kann dies in jeweils gleichwertige Weise durch Aufnahme einer buccalen Lösung aus einem flexiblen, 1 bis 5 ml fassenden Polyrmerbehältnis in die Mundhöhle erfolgen, oder durch die Aufnahme einer im Speichel schnell löslichen Galanthamin enthaltenden, biokompatiblen festen Matrix, etwa im Format einer Briefmarke, oder durch Einsprühen bzw. Eintropfen entsprechend formulierter Lösungen in die Nase, unter Verwerdung von flexiblen Kunststoffbehältnissen mit entsprechend geformtem Düsen. Als feste buccale Darreichungsform eignen sich insbesondere die in DE 199 13 731 oder DE 196 52 188 beschriebenen. Pernasale Formulierungen, die sich insbesondere für Alkaloide eignen, sind dem Fachmann ebenso vierfach bekannt, so etwa für Morphin durch die WO 00/76506.

## Patentansprüche

1. Medikament zur Anwendung in einem Verfahren zur Behandlung des Suchtverlangens, **dadurch gekennzeichnet, dass** das Medikament aus einer Kombination von zwei Darreichungsformen besteht, wobei die eine Darreichungsform ein transdermales therapeutisches System ist, das zumindest einen aus Galanthamin und pharmakologisch akzeptablen Salzen des Galanthamins bestehenden Gruppe ausgewählten Modulator nikotinischer Rezeptoren kontinuierlich abgibt, und die andere Darreichungsform, die unabhängig von der erstgenannten Darreichungsform verabreichbar ist, ein schnelles Eintreten von Galanthamin oder einem seiner pharmakologisch akzeptablen Salze in das Zentralnervensystem ermöglicht, wobei die einen schnellen Eintritt von Galanthamin oder einem pharmakologisch akzeptablen Salz des Galanthamins in das Zentralnervensystem ermöglichende Darreichungsform aus der Gruppe ausgewählt ist, die aus festen, im Speichel schnell löslichen, biokompatiblen Matrices, buccalen Lösungen sowie Sprüh- und Tropflösungen besteht.

2. Medikament zur Anwendung nach Anspruch 1, **dadurch gekennzeichnet, dass** das den Modulator oder die Modulatoren nikotinischer Rezeptoren kontinuierlich abgebende transdermale therapeutische System pro Tag zwischen 10 mg und 25 mg Galanthamin oder eines pharmakologisch akzeptablen Salzes von Galanthamin pro Tag abgibt.

3. Medikament zur Anwendung nach Anspruch 1 oder 2, **dadurch gekennzeichnet, dass** die einen schnellen Eintritt von Galanthamin oder einem pharmakologisch akzeptablen Salz des Galanthamins in das Zentralnervensystem ermöglichende Darreichungsform Galanthamin oder ein pharmakologisch akzeptables Salz des Galanthamins in einer Henge von 1 bis 5 mg enthält.

4. Medikament zur Anwendung nach Anspruch 1, **dadurch gekennzeichnet, dass** die einen schnellen Eintritt von Galanthamin oder einem pharmakologisch akzeptablen Salz des Galanthamins in das Zentralnervensystem ermöglichende Darreichungsform für Lösungen ein flexibles, zwischen 1 und 5 ml fassendes Kunststoffbehältnis ist.

5. Medikament zur Anwendung nach Anspruch 4, **dadurch gekennzeichnet, dass** das Kunststoffbehältnis mit Düsen versehen ist, durch die die Lötung in die Nase eingesprüht oder eingetropft werden kann.

## Claims

1. Medicament for use in a method for treating addiction craving, **characterized in that** said medicament consists of a combination of two dosage forms, one of said dosage forms being a transdermal therapeutic system which continuously releases at least one modulator of nicotinic receptors selected from the group consisting of galanthamine and pharmacologically acceptable salts of galanthamine, and the other dosage form, which can be administered independently from the first-mentioned dosage form, enabling a rapid entry of galanthamine or of one of its pharmacologically acceptable salts into the central nervous system, whereby the dosage form enabling a rapid entry of galanthamine or of a pharmacologically acceptable salt of galanthamine into the central nervous system is selected from the group consisting of solid, biocompatible matrices which are rapidly soluble in saliva, buccal solutions, as well as spray or drip solutions.

2. Medicament for the use according to claim 1, **characterised in that** the transdermal therapeutic system continuously releasing the modulator or modulators of nicotinic receptors releases between 10 mg and 25 mg of galanthamine or a pharmacologically acceptable salt of galanthamine, per day.

3. Medicament for the use according to claim 1 or 2, **characterised in that** the dosage form enabling a rapid entry of galanthamine or of a pharmacologically acceptable salt of galanthamine into the central nervous system contains galanthamine or a pharmacologically acceptable salt of galanthamine in an amount of from 1 to 5 mg.

4. Medicament for the use according to claim 1, **characterised in that** the dosage form for solutions which enables a rapid entry of galanthamine or a pharmacologically acceptable salt of galanthamine into the central nervous system is a flexible plastic container with a capacity of between 1 and 5 ml.

5. Medicament for the use according to claim 4, **characterized in that** the plastic container is provided with nozzles through which the solution can be sprayed or dripped into the nose.

## Revendications

1. Médicament à appliquer dans un procédé pour traiter l'état de manque dû à la toxicomanie, **caractérisé en ce que** le médicament constitué d'une combinaison de deux formes médicamenteuses, une forme médicamenteuse représentant un système thérapeutique transdermique qui libère en continu au moins un modulateur des récepteurs nicotiniques, choisi parmi le groupe constitué par la galanthamine et par des sels pharmacologiquement acceptables de la galanthamine, et l'autre forme médicamenteuse, qui peut être administrée indépendamment de la forme médicamenteuse mentionnée en premier lieu, permet une introduction rapide de la galanthamine ou d'un de ses sels pharmacologiquement acceptables dans le système nerveux central, la forme médicamenteuse qui permet une introduction rapide de la galanthamine ou d'un de ses sels pharmacologiquement acceptables dans le système nerveux central étant choisie parmi le groupe qui est constitué par des matrices biocompatibles solides, qui se dissolvent rapidement dans la salive, des solutions buccales, ainsi que des solutions pour nébulisation et des solutions goutte à goutte.

2. Médicament pour l'application selon la revendication 1, **caractérisé en ce que** le système thérapeutique transdermique libérant en continu le modulateur ou les modulateurs des récepteurs nicotiniques libère par jour entre 10 mg et 25 mg de galanthamine ou d'un sel pharmacologiquement acceptable de galanthamine.

3. Médicament pour l'application selon la revendication 1 ou 2, **caractérisé en ce que** la forme médicamenteuse qui permet une introduction rapide de galanthamine ou d'un de ses sels pharmacologiquement acceptables dans le système nerveux central contient de la galanthamine ou un sel pharmacologiquement acceptable de la galanthamine en une quantité de 1 à 5 mg.

4. Médicament pour l'application selon la revendication 1, **caractérisé en ce que** la forme médicamenteuse qui permet une introduction rapide de galanthamine ou d'un de ses sels pharmacologiquement acceptables dans le système nerveux central représente un récipient en matière synthétique flexible, destiné à des solutions, possédant une contenance entre 1 et 5 ml.

5. Médicament pour l'application selon la revendication 4, **caractérisé en ce que** le récipient en matière synthétique est muni de buses par lesquelles la solution peut être introduite dans le nez par nébulisation ou par goutte à goutte.
